(19) Europäisches Patentamt
European Patent Office
Office européen des brevets

(11) EP 4 324 813 B1

(12) EUROPEAN PATENT SPECIFICATION

(45) Date of publication and mention of the grant of the patent:
**01.07.2026 Bulletin 2026/27**

(21) Application number: **22788063.0**

(22) Date of filing: **30.03.2022**

(51) International Patent Classification (IPC):
***C07C 29/149*** (2006.01) ***C07C 31/27*** (2006.01)
***C07C 67/31*** (2006.01) ***C07C 69/757*** (2006.01)
***B01J 21/04*** (2006.01) ***B01J 23/80*** (2006.01)

(52) Cooperative Patent Classification (CPC):
(C-Sets available)
**C07C 29/149; B01J 21/04; B01J 23/80;**
**C07C 67/31;** B01J 23/002; B01J 23/005;
C07C 2603/86 (Cont.)

(86) International application number:
**PCT/JP2022/016217**

(87) International publication number:
**WO 2022/220138 (20.10.2022 Gazette 2022/42)**

(54) **PRODUCTION METHOD FOR PRODUCING DIMETHANOL COMPOUND HAVING NORBORNANE SKELETON**

HERSTELLUNGSVERFAHREN ZUR HERSTELLUNG EINER DIMETHANOLVERBINDUNG MIT NORBORNANGERÜST

PROCÉDÉ DE PRODUCTION POUR LA FABRICATION DE COMPOSÉ DIMÉTHANOL POSSÉDANT UN SQUELETTE DE NORBORNANE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2021 JP 2021067254**

(43) Date of publication of application:
**21.02.2024 Bulletin 2024/08**

(73) Proprietor: **MITSUBISHI GAS CHEMICAL COMPANY, INC.**
**Chiyoda-ku**
**Tokyo 100-8324 (JP)**

(72) Inventor: **KITAMURA, Mitsuharu**
**Tokyo 125-8601 (JP)**

(74) Representative: **Vossius & Partner**
**Patentanwälte Rechtsanwälte mbB**
**Siebertstrasse 3**
**81675 München (DE)**

(56) References cited:
**EP-A1- 0 657 214**
**EP-A1- 3 202 815**
**WO-A1-2015/147242**
**WO-A1-2016/052370**
**JP-A- H0 236 135**
**US-A1- 2017 088 504**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

(52) Cooperative Patent Classification (CPC): (Cont.)

C-Sets
**C07C 29/149, C07C 31/278;**
**C07C 67/31, C07C 69/757**

## Description

### Technical Field

The present invention relates to a production method for producing a dimethanol compound having a norbornane skeleton.

### Background Art

A dimethanol compound having a norbornane skeleton is known to exhibit excellent features when used as an adhesive or a resin raw material. As for a method for producing such a dimethanol compound having a norbornane skeleton, for example, Patent Literature 1 describes that the dimethanol compound having a norbornane skeleton is obtained through the hydrogenation reaction of a corresponding ester compound.

Many methods have been proposed since 1930s as methods for producing an aliphatic alcohol by the hydrogenation of an aliphatic ester compound. For example, a copper-based catalyst mainly containing copper oxide is known as a catalyst for use in hydrogenation reaction, and in the presence of this catalyst, hydrogenation reaction can be carried out by reacting an ester compound with a hydrogen gas. Activation conditions for such a catalyst are determined in consideration of a form of use, a use method, a reaction scheme, etc. For example, in the case of adopting a fixed-bed reaction scheme, a gas-phase reduction method is adopted for the reductive activation of a shaped catalyst. Patent Literature 2 describes that, for avoiding local overheat due to rapid catalyst reduction, it is desired to carefully perform catalyst reduction at a predetermined temperature under distribution of an inert gas having a hydrogen concentration of several percents to several tens of percents.

On the other hand, in the case of adopting a suspended-bed reaction scheme, a catalyst form is a powder. Patent Literature 3 describes that in the case of activating a catalyst, either a gas-phase reduction method or a liquid-phase reduction method which is performed in a solvent such as hydrocarbons may be used.

### Citation List

### Patent Literature

Patent Literature 1: International Publication No. WO 2015/147242
Patent Literature 2: Japanese Patent Laid-Open No. 61-161146
Patent Literature 3: Japanese Patent Laid-Open No. 1-305042

### Summary of Invention

### Technical Problem

In the case of adopting a fixed-bed reaction scheme to carry out hydrogenation reaction, as described in Patent Literature 2, it is reported that it requires even 4 to 14 days for catalyst reduction, leading to problems with the productivity of an aliphatic alcohol. Furthermore, it is concerned that an ester compound remains in a reaction liquid when operation is disturbed or catalytic activity is lowered. Particularly, when a target compound is a dimethanol compound having a norbornane skeleton, the target compound and its intermediate both have a high viscosity and a high boiling point and are therefore difficult to separate by simple distillation or thin-membrane distillation. On the other hand, use of distillation purification at a large number of stages for the separation of the target compound (dimethanol compound) compromises yield. Thus, there is a room for improvement from an economic viewpoint.

In the case of adopting a suspended-bed reaction scheme to carry out hydrogenation reaction, as described in Patent Literature 3, it is reported that use of a catalyst of copper oxide and zinc oxide exhibits very high activity even under reaction conditions of a low temperature and a low pressure. In this technique, however, it is concerned that a large amount of an intermediate remains in a target compound, and the separation therebetween is not easy as mentioned above.

In Patent Literature 1, a substrate ester compound, a solvent, and a catalyst are charged and then catalyst activation and hydrogenation reaction are performed in one pot, and mild conditions involving a reaction temperature of 215°C, a reaction pressure of 10 MPa, and a reaction time of 8 hours are described therein. However, studies of the present inventors have revealed that a significant amount of an intermediate remains in the resulting reaction liquid. When the produced target compound is used as a polymer raw material in a state where the aforementioned amount of the intermediate is remained in the target compound, the intermediate tends to inhibit reaction into the polymer. Hence,

distillation purification at a large number of stages is required for separating the target compound and the intermediate, as in the case of Patent Literature 2. This distillation purification would compromise yield, and thus, there is still room for improvement from the economic viewpoint.

It may be possible to increase the purity of a target compound by extending a reaction time or by discarding a low-molecular alcohol as a by-product of ester hydrogenolysis, together with a hydrogen gas, for the purpose of increasing a hydrogen gas concentration in a gas phase. However, it cannot be said that such a production method is an optimum method from the viewpoint of productivity, a loss of a hydrogen gas, etc.

The present invention has been made in light of the problems described above. An object of the present invention is to provide a method for producing a dimethanol compound having a norbornane skeleton which can be efficiently carried out hydrogenation reaction and can reduce the amount of an intermediate remained in the target compound.

Solution to Problem

The present inventors have found that the problem can be solved by removing water as a by-product of hydrogenation reaction from the reaction system in the production of a dimethanol compound having a norbornane skeleton, and thus have completed the present invention.

Specifically, the present invention is as follows.

[1] A production method for producing a target compound represented by the following formula (1), the production method comprising:

a step (A) of subjecting a mixed liquid comprising a starting compound represented by the following formula (2) and a solvent to a hydrogenation reduction in a presence of a catalyst having hydrogenation ability,
wherein, in the step (A), water is removed from a reaction system and a water content of the mixed liquid measured after the removal of water is 4500 ppm or less:

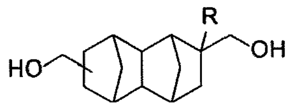

(1)

wherein R represents H, $CH_3$, or $C_2H_5$,

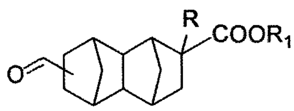

(2)

wherein R represents H, $CH_3$, or $C_2H_5$, and $R_1$ represents $CH_3$, $C_2H_5$, $C_3H_7$, or $C_4H_9$.

[3] The production method according to [1], wherein, in the step (A), the catalyst is activated at a first temperature and a first pressure, and after the activation, the starting compound is hydrogenated at a second temperature and a second pressure.
[4] The production method according to [3], wherein, before the hydrogenation, the removal of water is performed.
[5] The production method according to [3] or [4], wherein the first temperature and the first pressure are 100°C or higher and 170°C or lower and 0.5 MPa or higher and 5 MPa or lower, respectively, and
the second temperature and the second pressure are higher than 170°C and 250°C or lower and higher than 5 MPa and 20 MPa or lower, respectively.
[6] The production method according to any of [1] or [3] to [5], wherein a content of an intermediate represented by the following formula (3) in the target compound is 0.5% by mass or less:

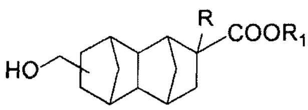

(3)

wherein R and $R_1$ are as defined in the formula (2).

## Advantageous Effects of Invention

The present invention can provide a method for producing a dimethanol compound having a norbornane skeleton which can efficiently carry out hydrogenation reaction and can reduce the amount of an intermediate remained in the target compound.

## Description of Embodiments

Hereinafter, an embodiment for carrying out the present invention (hereinafter, also referred to as the "present embodiment") will be described in detail. The present embodiment given below is an example for explaining the present invention and does not intend to limit the present invention to the contents given below. The present invention can be carried out through appropriate changes or modifications without departing from the scope of the present invention. In the present specification, the term "to" means that numeric values described before and after the term are included as upper limit and lower limit values, unless otherwise specified.

<Method for producing dimethanol compound having norbornane skeleton>

The production method of the present embodiment is a method for producing a target compound represented by the following formula (1) (i.e., a dimethanol compound having a norbornane skeleton, represented by the formula (1); hereinafter, also simply referred to as a "target compound"), the production method comprising:

a step (A) of subjecting a mixed liquid containing a starting compound represented by the following formula (2) and a solvent to hydrogenation reduction in the presence of a catalyst having hydrogenation ability,
wherein, in the step (A), water is removed from the reaction system:

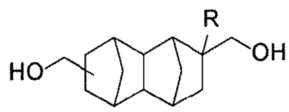

(1)

wherein R represents H, $CH_3$, or $C_2H_5$,

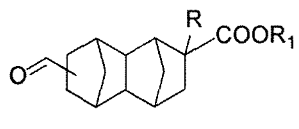

(2)

wherein R represents H, $CH_3$, or $C_2H_5$, and $R_1$ represents $CH_3$, $C_2H_5$, $C_3H_7$, or $C_4H_9$.

The production method of the present embodiment is configured as described above and as such, can efficiently carry out hydrogenation reaction in producing the target compound and can reduce the amount of an intermediate remained in the target compound. The target compound can be preferably used as a paint additive, an adhesive, a resin raw material, or the like.

A synthesis route for producing the target compound can adopt, for example, but not limited to, a synthesis route shown in the following formula (I) using dicyclopentadiene or cyclopentadiene and olefin having a functional group as starting materials:

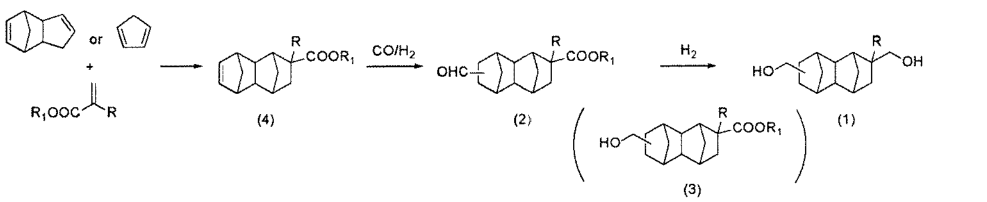

(4) (2) (1) (3)

(I)

wherein R in the formulas (1) to (4) represents H, $CH_3$, or $C_2H_5$, and $R_1$ in the formulas (1) to (3) represents $CH_3$, $C_2H_5$, $C_3H_7$, or $C_4H_9$.

The formula (3) in the formula (I) represents a synthetic intermediate capable of being entrained with the target compound. Hereinafter, this synthetic intermediate is also simply referred to as an "intermediate" for the sake of convenience of description.

[Monoolefin having 14 to 19 carbon atoms represented by formula (4)]

The monoolefin having 14 to 19 carbon atoms represented by the following formula (4) according to the present embodiment can be produced through the Diels-Alder reaction of olefin having a functional group with dicyclopentadiene:

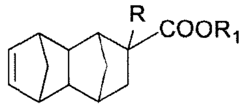

(4)

wherein R represents H, $CH_3$, or $C_2H_5$, and $R_1$ represents $CH_3$, $C_2H_5$, $C_3H_7$, or $C_4H_9$.

Examples of the olefin having a functional group for use in the Diels-Alder reaction include, but are not particularly limited to, methyl methacrylate, ethyl methacrylate, propyl methacrylate, butyl methacrylate, methyl acrylate, ethyl acrylate, propyl acrylate, and butyl acrylate. Among them, methyl methacrylate, ethyl methacrylate, methyl acrylate, and ethyl acrylate are preferred.

The dicyclopentadiene for use in the Diels-Alder reaction of the present embodiment preferably has a high purity and preferably has a lower content of impurities such as butadiene and isoprene. The purity of the dicyclopentadiene is 90% or more, more preferably 95% or more. It is known that dicyclopentadiene is depolymerized into cyclopentadiene (so-called monocyclopentadiene) under heating conditions. Therefore, cyclopentadiene may be used instead of the dicyclopentadiene. It is considered that the monoolefin having 14 to 19 carbon atoms represented by the formula (4) is substantially produced via monoolefin having 9 to 14 carbon atoms represented by the formula (5) given below (first-stage Diels-Alder reaction product). It is also considered that the produced monoolefin of the following formula (5) undergoes, as a new parent diene compound (dienophile), Diels-Alder reaction (second-stage Diels-Alder reaction) with cyclopentadiene (diene) present in the reaction system to produce the monoolefin having 14 to 19 carbon atoms represented by the formula (4) :

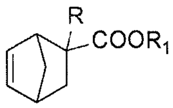

(5)

wherein R represents H, $CH_3$, or $C_2H_5$, and $R_1$ represents $CH_3$, $C_2H_5$, $C_3H_7$, or $C_4H_9$.

In the presence of cyclopentadiene in the reaction system, the Diels-Alder reaction at two sages tends to proceed efficiently. Therefore, the reaction temperature of the Diels-Alder reaction is preferably 100°C or higher, more preferably 120°C or higher, further preferably 130°C or higher. On the other hand, it is preferred to perform the reaction at a temperature of 250°C or lower for suppressing the formation of a high-boiling substance as a by-product. Hydrocarbons, alcohols, esters, or the like may be used as reaction solvents, and aliphatic hydrocarbons having 6 or more carbon atoms, cyclohexane, toluene, xylene, ethylbenzene, mesitylene, propanol, butanol, or the like is suitable.

Diverse reaction schemes such as a batch system using a tank-type reactor or the like, a semi-batch system in which a substrate or a substrate solution is supplied to a tank-type reactor under reaction conditions, and a continuous distribution system in which substrates are distributed to a tube-type reactor under reaction conditions can be adopted as reaction schemes of the Diels-Alder reaction.

The reaction product (monoolefin having 14 to 19 carbon atoms represented by the formula (4)) obtained through the Diels-Alder reaction may be directly used as a starting material for subsequent hydroformylation reaction or may be subjected to the next step after being purified by a method such as distillation, extraction, or crystal precipitation. The monoolefin having 14 to 19 carbon atoms represented by the formula (4) is not limited to those synthesized as mentioned above, and a commercially available product, if obtainable, may be used in the production method of the present embodiment.

[Starting compound represented by formula (2)]

The starting compound represented by the formula (2) in the formula (I) can be produced, for example, by subjecting the monoolefin having 14 to 19 carbon atoms represented by the formula (4) and carbon monoxide and hydrogen gases to hydroformylation reaction in the presence of a rhodium compound and an organic phosphorus compound.

The rhodium compound for use in the hydroformylation reaction is not particularly limited, and, for example, a compound that forms a complex with an organic phosphorus compound and exhibits hydroformylation activity in the presence of carbon monoxide and hydrogen, can be used. A precatalyst such as dicarbonyl(acetylacetonato)rhodium (hereinafter, also referred to as "$Rh(acac)(CO)_2$"), $Rh_2O_3$, $Rh_4(CO)_{12}$, $Rh_6(CO)_{16}$, or $Rh(NO_3)_3$ may be introduced together with an organic phosphorus compound into a reaction mixture to form a rhodium-metal carbonyl hydride-phosphorus complex having catalytic activity in a reaction container, or a rhodium-metal carbonyl hydride-phosphorus complex may be prepared in advance and introduced into a reactor. In the present embodiment, it is preferred that $Rh(acac)(CO)_2$ should be reacted with an organic phosphorus compound in the presence of a solvent and then introduced together with an excess of an organic phosphorus compound into a reactor to form a rhodium-organic phosphorus complex having catalytic activity, which is used in the hydroformylation reaction.

The amount of the rhodium compound used in the hydroformylation reaction is preferably 0.1 to 60 μmol, more preferably 0.1 to 30 μmol, further preferably 0.2 to 20 μmol, still further preferably 0.5 to 10 μmol, based on 1 mol of the monoolefin having 14 to 19 carbon atoms represented by the formula (4) which is a substrate of the hydroformylation reaction. When the amount of the rhodium compound used is less than 60 μmol based on 1 mol of the monoolefin having 14 to 19 carbon atoms, rhodium catalyst cost can be reduced without providing recovery and recycling facilities of a rhodium complex. Therefore, economic burdens associated with recovery and recycling facilities can be reduced.

In the hydroformylation reaction, examples of the organic phosphorus compound that forms a catalyst of the hydroformylation reaction with the rhodium compound include phosphine represented by the general formula $P(-R_1)(-R_2)(-R_3)$ and phosphite represented by the general formula $P(-OR_1)(-OR_2)(-OR_3)$. In these general formulas, specific examples of $R_1$, $R_2$, and $R_3$ include an aryl group optionally substituted by an alkyl group or an alkoxy group having 1 to 4 carbon atoms, and an alicyclic alkyl group optionally substituted by an alkyl group or an alkoxy group having 1 to 4 carbon atoms. Triphenylphosphine or triphenyl phosphite is suitably used. The amount of the organic phosphorus compound used is preferably 300 times to 10000 times, more preferably 500 times to 10000 times, further preferably 700 times to 5000 times, still further preferably 900 times to 2000 times the mol of a rhodium atom in the rhodium compound. When the amount of the organic phosphorus compound used is 300 or more times the mol of a rhodium atom, a rhodium-metal carbonyl hydride-phosphorus complex as a catalytically active substance contributes to stability. As a result, reaction efficiency tends to be improved. When the amount of the organic phosphorus compound used is 10000 or less times the mol of a rhodium atom, economic performance tends to be improved from the viewpoint of cost required for the organic phosphorus compound.

The hydroformylation reaction may be performed without the use of a solvent and can be preferably carried out by using a solvent inert to the reaction. The solvent is not particularly limited as long as the solvent dissolves the monoolefin having 14 to 19 carbon atoms represented by the formula (3), the dicyclopentadiene or the cyclopentadiene, the rhodium compound, and the organic phosphorus compound. Specific examples thereof include hydrocarbons such as aliphatic hydrocarbon, alicyclic hydrocarbon, and aromatic hydrocarbon, esters such as aliphatic ester, alicyclic ester, and aromatic ester, alcohols such as aliphatic alcohol and alicyclic alcohol, and other solvents such as aromatic halide. Among them, hydrocarbons are preferred. Among the hydrocarbons, alicyclic hydrocarbon and aromatic hydrocarbon are preferred.

A temperature at which the hydroformylation reaction is performed is preferably 40°C to 160°C, more preferably 80°C to 140°C. When the reaction temperature is 40°C or higher, a sufficient reaction rate is obtained and prevents the starting material monoolefin from remaining. When the reaction temperature is 160°C or lower, the formation of by-products derived from the starting material monoolefin or a reaction product is suppressed. This tends to be able to prevent reduction in reaction performance.

In the case of performing the hydroformylation reaction, the reaction is performed under pressurization with carbon monoxide (hereinafter, also referred to as "CO") and hydrogen (hereinafter, also referred to as "$H_2$") gases. The CO and $H_2$ gases may be each independently introduced into the reaction system or may be introduced as a mixed gas prepared in advance into the reaction system. The molar ratio between the CO and $H_2$ gases (= $CO/H_2$) to be introduced into the reaction system is preferably 0.2 to 5, more preferably 0.5 to 2, further preferably 0.8 to 1.2. When the molar ratio between the CO and $H_2$ gases falls within the range described above, the reaction activity of the hydroformylation reaction and selectivity to targeted aldehyde tend to be improved. The amount of the CO and $H_2$ gases introduced into the reaction system decreases as the reaction proceeds. Therefore, use of a mixed gas of CO and $H_2$ prepared in advance may facilitate reaction control.

The reaction pressure of the hydroformylation reaction is preferably 1 to 12 MPa, more preferably 1.2 to 8 MPa, further preferably 1.5 to 5 MPa. When the reaction pressure is 1 MPa or higher, a sufficient reaction rate is easily obtained

and tends to be able to prevent the starting material monoolefin from remaining. When the reaction pressure is 12 MPa or lower, an expensive facility excellent in pressure resistance is no longer necessary. This tends to be economically advantageous. Particularly, in the case of performing the reaction in a batch system or a semi-batch system which involves depressurization by the discharge of the CO and $H_2$ gases after the completion of reaction, a lower pressure causes a smaller loss of the CO and $H_2$ gases. This tends to be economically advantageous.

The reaction scheme in performing the hydroformylation reaction is preferably batch reaction or semi-batch reaction. The semi-batch reaction can be performed by adding the rhodium compound, the organic phosphorus compound, and the solvent to a reactor, creating the aforementioned reaction conditions through pressurization with $CO/H_2$ gases, heating, or the like, and then supplying the starting material monoolefin or its solution to the reactor.

The reaction product obtained through the hydroformylation reaction may be directly used as a starting material for subsequent reduction reaction or may be subjected to the next step after being purified by for example, distillation, extraction, or crystal precipitation. The starting compound represented by the formula (2) is not limited to those synthesized as mentioned above, and a commercially available product, if obtainable, may be used in the production method of the present embodiment.

[Step (A)]

The production method of the present embodiment comprises a step (A) of subjecting a mixed liquid containing a starting compound represented by the formula (2) and a solvent to hydrogenation reduction in the presence of a catalyst having hydrogenation ability (hereinafter, also referred to as a "hydrogenation catalyst"). In the present embodiment, in this step (A), water is removed from the reaction system, whereby not only is the target compound obtained from the starting compound, but the amount of an intermediate remained in the target compound is reduced. This is presumably because, while water is produced as a by-product in the reaction system of the step (A), this water probably inactivates the activated hydrogenation catalyst, though this inference does not intend to limit the mechanism of action of the present embodiment. In the present embodiment, the removal of water facilitates maintaining the activity of the hydrogenation catalyst. As a result, the reaction proceeds sufficiently, presumably reducing the amount of an intermediate remained in the target compound.

Examples of the hydrogenation catalyst for use in the step (A) include, but are not limited to, catalysts containing at least one element selected from the group consisting of copper, chromium, iron, zinc, and aluminum. Among them, copper-based catalysts such as a Cu-Cr catalyst, a Cu-Zn catalyst, a Cu-Zn-Al catalyst, and a Cu-Fe-Al catalyst are preferred, and a Cu-Cr catalyst and a Cu-Zn-Al catalyst are more preferred.

The hydrogenation catalyst mentioned above may be obtained as a commercially available product. Examples thereof include E-01X (Cu-Zn-Al catalyst; the amount of copper oxide: 46%, the amount of zinc oxide: 46%, the amount of aluminum oxide: 5%) and N-203S (the amount of copper oxide: 46.1%, the amount of chromium oxide: 43.8%, the amount of manganese oxide: 4.2%) manufactured by JGC C&C.

The amount of the hydrogenation catalyst used is not particularly limited and is preferably 1 to 50% by mass based on the starting compound represented by the formula (2) which is a substrate. When the amount of the hydrogenation catalyst used is 1% by mass or more, the reaction proceeds sufficiently. As a result, the yield of the target compound tends to be improved. Even if the amount of the hydrogenation catalyst used is more than 50% by mass, a reaction rate improving effect commensurate with the amount of the catalyst subjected to the reaction tends to be not obtained. Hence, it is preferred from an economic standpoint to set the amount of the hydrogenation catalyst used to 50% by mass or less. Specifically, when the amount of the hydrogenation catalyst used falls within the range mentioned above, the hydrogenation reaction tends to be able to be efficiently carried out. From the viewpoint described above, the amount of the hydrogenation catalyst used is more preferably 2 to 20% by mass, further preferably 5 to 10% by mass.

In the step (A), a solvent is used. Examples of the solvent include, but are not limited to, aliphatic hydrocarbons, alicyclic hydrocarbons, aromatic hydrocarbons, and alcohols. Among them, alicyclic hydrocarbons, aromatic hydrocarbons, and alcohols are preferably used as solvents. Preferred specific examples of these solvents include cyclohexane, toluene, xylene, methanol, ethanol, 1-propanol, cyclohexanol, and 2-octanol.

In the present embodiment, the water content of the mixed liquid measured after the removal of water is preferably 4500 ppm or less, more preferably 4000 ppm or less, further preferably 3000 ppm or less, from the viewpoint of more reducing the amount of an intermediate remained in the target compound.

The water content can be measured on the basis of a method described in Examples mentioned later.

The amount of a by-product water in the reaction system can be predicted from the amount of copper oxide in the catalyst when a copper-based catalyst is taken as an example of the hydrogenation catalyst. Specifically, the water content can be predicted as a theoretical value according to the following expression.

Water content = Amount of the catalyst (g) $\times$ Copper oxide content (% by mass) / 100 $\times$ 18.015 (water molecular weight) / 79.545 (copper oxide molecular weight).

It is preferred to adjust the amount of water removed on the basis of the predicted value such that the water

content of the mixed liquid falls within the range mentioned above.

In the present embodiment, it is preferred that in the step (A), the catalyst should be activated at a first temperature and a first pressure, and after the activation, the starting compound should be hydrogenated at a second temperature and a second pressure, from the viewpoint of more enhancing reaction efficiency. As mentioned above, a by-product water probably inactivates the activated hydrogenation catalyst. It is therefore preferred to perform the removal of water before hydrogenation.

The activation of the hydrogenation catalyst is an operation for reducing the hydrogenation catalyst and can be carried out at a first temperature and a first pressure.

In the present embodiment, the first temperature is preferably 100°C or higher and 170°C or lower. When the first temperature is 170°C or lower, side reaction or decomposition reaction tends to be prevented. When the first temperature is 100°C or higher, the reduction reaction of the catalyst tends to be completed in a moderate time. From a similar viewpoint, the first temperature is more preferably 120 or higher and 170°C or lower.

In the present embodiment, the first pressure is preferably 0.5 MPa or higher and 5 MPa or lower. When the first pressure is 0.5 MPa or higher, a sufficient reaction rate tends to be obtained. When the first pressure is 5 MPa or lower, a loss of a $H_2$ gas is reduced in removing water. This tends to be economically advantageous. From a similar viewpoint, the first pressure is more preferably 1 MPa or higher and 3 MPa or lower, further preferably 1.5 MPa or higher and 2 MPa or lower.

In the present embodiment, the reaction time for the activation of the hydrogenation catalyst is preferably 0.2 hours or longer and 3 hours or shorter. When the first reaction time is 0.2 hours or longer, the reduced state of the catalyst tends to be more favorable. When the first reaction time is 3 hours or shorter, the reaction time tends to be able to be decreased while the reduction reaction of the catalyst is allowed to proceed sufficiently.

The activation of the hydrogenation catalyst may be carried out in the absence of the starting compound. In the present embodiment, it is preferred to carry out the activation in the presence of the starting compound, from the viewpoint of reaction efficiency. In the case of carrying out the activation of the hydrogenation catalyst in the presence of the starting compound, water is produced as a by-product reduced water. An operation for removing such water is not particularly limited, and, for example, any of the following methods can be used: a method of discharging water present in a gas-phase moiety to the outside of the reaction system by purging the gas-phase moiety several times; and a method of discharging water to the outside of the reaction system by placing a nozzle line in the mixed liquid in advance and bubbling a $H_2$ gas or an inert gas such as $N_2$. The completion of the treatment can be determined by using a method of placing an exhaust gas trap and continuing an operation with two to three times the expected amount of water as a guideline, or a method of measuring a water content value in a reaction liquid.

The hydrogenation of the starting compound is an operation for obtaining the target compound and can be carried out at a second temperature and a second pressure.

In the present embodiment, the second temperature is preferably higher than 170°C and 250°C or lower. When the second temperature is 250°C or lower, the target compound tends to be obtained at a high yield by preventing side reaction or decomposition reaction. When the second temperature is higher than 170°C, the reaction tends to be completed in a moderate time. Thus, reduction in productivity or reduction in target compound yield tends to be able to be circumvented. From a similar viewpoint, the second temperature is more preferably 190°C or higher and 230°C or lower.

In the present embodiment, the second pressure is preferably higher than 5 MPa and 20 MPa or lower. When the second pressure is 20 MPa or lower, the target compound tends to be obtained at a high yield by preventing side reaction or decomposition reaction. When the second pressure is higher than 5 MPa, the reaction tends to be completed in a moderate time. From a similar viewpoint, the second pressure is more preferably higher than 5 MPa and 15 MPa or lower, further preferably higher than 5 MPa and 12 MPa or lower.

In the hydrogenation according to the present embodiment, a gas inert to the hydrogenation reaction (e.g., nitrogen or argon) may be allowed to coexist.

The pressures mentioned above mean values as hydrogen partial pressures.

In the present embodiment, the content of the intermediate represented by the formula (3) in the target compound obtained in the step (A) according to the present embodiment is preferably 0.5% by mass or less, from the viewpoint of physical properties to be obtained when the target compound is further processed. The content of the intermediate can be measured on the basis of a method described in Examples mentioned later. The content of the intermediate can be adjusted to the range described above by the amount of water removed in the step (A).

The target compound obtained in the step (A) according to the present embodiment can be purified by, for example, distillation, extraction, or crystal precipitation.

## Examples

Hereinafter, the present embodiment will be described in more detail with reference to Examples. However, the

present embodiment is not limited by these Examples.

<Analysis method>

(1) Measurement conditions for gas chromatography analysis

Analyzer: Capillary gas chromatograph GC-2010 Plus manufactured by Shimadzu Corp.
Analysis column 1: InertCap 1 (30 m, 0.32 mm I.D., thickness: 0.25 μm) manufactured by GL Sciences Inc.
Oven temperature 1: 60°C (0.5 min)-15°C/min-280°C (4 min)
Detector 1: FID, temperature: 280°C
Internal standard: p-Xylene
Analysis column 2: InertCap WAX (30 m, 0.32 mm I.D., thickness: 0.25 μm) manufactured by GL Sciences Inc.
Oven temperature 2: 60°C (0.5 min)-20°C/min-250°C (20 min) Detector 2: FID, temperature: 250°C

(2) GC-MS measurement conditions

Analyzer: GCMS-QP2010 Plus manufactured by Shimadzu Corp. Ionization voltage: 70 V
Analysis column: DB-1 (30 m, 0.32 mm I.D., thickness: 1.00 μm) manufactured by Agilent Technologies, Inc.
Oven temperature: 60°C (0.5 min)-15°C/min-280°C (4 min)

(3) Water content measurement conditions

Analyzer: Coulometric KF water content meter 899
Coulometer manufactured by Metrohm AG

(4) Weight average molecular weight
A calibration curve was prepared from polystyrene standards having known molecular weights (molecular weight distribution = 1) using gel permeation chromatography (GPC) and tetrahydrofuran as a developing solvent. On the basis of this calibration curve, the weight average molecular weight was calculated from a retention time of GPC. The GPC measurement can specifically be carried out under the following conditions.

Apparatus: HLC-8320GPC manufactured by Tosoh Corp.
Column: Guard column: TSK guard column Super MPHZ-M × 1
Analysis column: TSKgel SuperMultipore HZ-M × 3 Solvent: Tetrahydrofuran
Injection volume: 10 μL
Sample concentration: 0.2 w/v% solution in tetrahydrofuran
Solvent flow rate: 0.35 ml/min
Measurement temperature: 40°C
Detector: RI

(5) Glass transition temperature
The glass transition temperature was measured with a differential scanning calorimeter (DSC). Specifically, the measurement was carried out at temperature increase and decrease rates of 10°C/min using DSC7000X manufactured by Hitachi High-Tech Science Corp. as the apparatus.

<Example 1>

The target compound was synthesized through the route shown in the following formula (Ia).

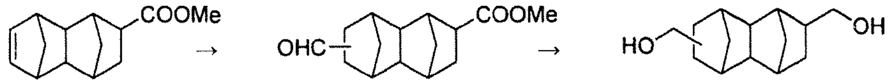

(4a) (2a) (1a) (I a)

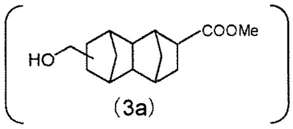

(3a)

(Obtainment of monoolefin)

215 g (2.50 mol) of methyl acrylate and 165 g (1.25 mol) of dicyclopentadiene were charged into a 500 mL stainless reactor, and were reacted at 220°C for 2 hours. A reaction liquid containing 152 g of monoolefin represented by the formula (4a) was obtained and subjected to distillation purification. Then, a portion of the purified product was used for the subsequent reaction.

(Obtainment of aldehyde)

A 500 mL stainless reactor was used to perform the hydroformylation reaction of the monoolefin represented by the formula (4a). To the reactor were added 100 g of the distillation-purified monoolefin represented by the formula (4a), 96 g of 2-octanol (manufactured by Ogura Gosei Kogyo Ltd.), 213 mg (1500 ppm by mol based on the monoolefin) of triphenyl phosphite (manufactured by FUJIFILM Wako Pure Chemical Corp.), and 355 μg (3 ppm by mol based on the monoolefin) of Rh(acac) $(CO)_2$ (manufactured by N.E. CHEMCAT Corp.). The atmosphere inside the reactor was replaced with nitrogen and a $CO/H_2$ mixed gas three times each. Then, the system was pressurized with a $CO/H_2$ mixed gas, followed by reaction at 100°C at 2 MPa for 3 hours.

After the completion of reaction, the reaction liquid was analyzed by gas chromatography and GC-MS. As a result, a reaction liquid containing 112.6 g of a starting compound represented by the formula (2a) having a molecular weight of 248 (rate of substrate conversion:

100% (= (Monoolefin charged - Remaining monoolefin charged) / Monoolefin charged) × 100%) was obtained as a main product with an aldehyde yield of 99.5% (= Main product / Monoolefin charged × 100%)).

Subsequently, the starting compound represented by the formula (2a) was subjected to distillation purification. Then, a portion of the purified product was subjected to the subsequent reaction.

(Reduction of catalyst and dehydration of reduced water)

To a 300 mL stainless reactor equipped with a nozzle for nitrogen bubbling were added 91.6 g of the distillation-purified starting compound represented by the formula (2a), 15.0 g of a Cu-Zn-Al catalyst (E-01X manufactured by JGC C&C), and 199.4 g of 2-octanol (manufactured by Ogura Gosei Kogyo Ltd.). The atmosphere inside the reactor was replaced with nitrogen and a $H_2$ gas three times each. Then, the system was pressurized with a $H_2$ gas, followed by the reduction of the catalyst at 140°C at 2 MPa for 1 hour. A trap was placed in an exhaust gas line. While the temperature was kept at 140°C, the pressure was dropped to 0 MPa. Then, a very small amount of a nitrogen gas was injected from the nozzle for nitrogen bubbling to remove reduced water in the reaction liquid. The amount of a liquid trapped was 5.05 g, and the water content therein was 1.41 g. The water concentration of the reaction liquid was 2743 ppm.

(Obtainment of dimethanol through hydrogenation reaction)

Subsequently, the atmosphere inside the reactor was replaced with a $H_2$ gas three times. Then, the system was pressurized with a $H_2$ gas, followed by hydrogenation reaction at 215°C at 9.5 MPa. During the reaction, sampling was performed every 5 hours.

The sampled liquid was diluted with acetone, and the catalyst was filtered off through a membrane filter having a pore size of 0.2 μm. Then, a target compound represented by the formula (1a) having a molecular weight of 222 as a main product, and an ester compound (intermediate) represented by the formula (3a) having a molecular weight of 250 were quantitatively analyzed by gas chromatography and GC-MS. This quantitative analysis was conducted using analysis column 2 (InertCap WAX). The amount of the intermediate represented by the formula (3a) 15 hours later was 0.36% (=

Intermediate represented by the formula (3a) / (All the components except for 2-octanol and acetone) × 100).

After the completion of reaction, the reaction liquid was analyzed by gas chromatography using analysis column 1 (InertCap 1) to confirm a reaction liquid containing 79.1 g of the target compound represented by the formula (1a) (rate of substrate conversion: 100% (= (Starting compound charged - Remaining starting compound) / Starting compound charged) × 100%) with a target compound yield of 96.5% (= Target compound / Starting compound charged × 100%)).

Further, the reaction liquid was subjected to simple distillation purification using a 300 mL three-neck recovery flask equipped with a thermometer, a nozzle for $N_2$ bubbling, and a stirring bar (the number of distillation stages: one stage, distillate temperature: 170 to 180°C, degree of vacuum: 270 Pa (2 Torr)).

The purified product was analyzed by gas chromatography using analysis column 1 (InertCap 1) to confirm 72.8 g of a main fraction containing the target compound represented by the formula (1a) with a dimethanol purity of 99.2% (= Target compound represented by the formula (1a) / (All the components except for acetone) × 100)) and a distillation yield of 92.0% (=Target compound obtained / Target compound charged × 100%)). Also, analysis was conducted using analysis column 2 (InertCap WAX). The amount of the intermediate represented by the formula (3a) was 0.35% (= Intermediate represented by the formula (3a) / (All the components except for 2-octanol and acetone) × 100).

Subsequently, 23.53 g (0.106 mol in terms of the target compound) of the main fraction, 23.02 g (0.107 mol) of diphenyl carbonate, and 0.07 mg (0.8 μmol) of sodium bicarbonate were placed in a 300 mL reactor with a stirrer and a distillatory, and transesterification reaction was performed in a nitrogen atmosphere to obtain a polycarbonate resin. The obtained polycarbonate resin had a weight average molecular weight (Mw) of 8,000 and a glass transition temperature (Tg) of 110°C.

<Comparative Example 1>

Reaction was performed by the same operation as in Example 1 except that the operation of dehydrating reduced water was not performed. The amount of the intermediate represented by the formula (3a) 25 hours later was as large as 1.00%. The water concentration of the reaction liquid was 7065 ppm.

After the completion of reaction, the reaction liquid was analyzed by gas chromatography using analysis column 1 (InertCap 1) to confirm a reaction liquid containing 77.5 g of the target compound represented by the formula (1a) (rate of substrate conversion: 100% (= (Starting compound charged - Remaining starting compound) / Starting compound charged) × 100%) with a dimethanol yield of 94.5% (= Target compound / Starting compound charged × 100%)).

Further, the reaction liquid was subjected to simple distillation purification using a 300 mL three-neck recovery flask equipped with a thermometer, a nozzle for $N_2$ bubbling, and a stirring bar (the number of distillation stages: one stage, distillate temperature: 170 to 180°C, degree of vacuum: 270 Pa (2 Torr)).

The purified product was analyzed by gas chromatography using analysis column 1 (InertCap 1) to confirm 69.0 g of a main fraction containing the target compound represented by the formula (1a) with a dimethanol purity of 98.9% (= Target compound represented by the formula (1a) / (All the components except for acetone) × 100)) and a distillation yield of 89.0% (=Target compound obtained / Target compound charged × 100%)). Also, analysis was conducted using analysis column 2 (InertCap WAX). The amount of the intermediate represented by the formula (3a) was 0.98% (= Intermediate represented by the formula (3a) / (All the components except for 2-octanol and acetone) × 100).

Since the dehydration operation was not performed, the effect of reducing the amount of the intermediate represented by the formula (3a) was low, and more than 0.5% of the intermediate was remained in the product.

Subsequently, transesterification reaction was performed in the same manner as in Example 1 to obtain a polycarbonate resin except that instead of the main fraction obtained in Example 1, the main fraction obtained as described above was used in the same amount as in Example 1. The obtained polycarbonate resin had a weight average molecular weight (Mw) of 6,800 and a glass transition temperature (Tg) of 105°C.

<Example 2>

Reaction was performed by the same operation as in Example 1 except that the amount of the hydrogenation catalyst was changed to 9.16 g. The amount of a liquid trapped was 3.01 g, and the water content therein was 0.85 g. The water concentration of the reaction liquid was 2585 ppm. The amount of the intermediate represented by the formula (3a) 25 hours after the start of the hydrogenation reaction was 0.44%.

After the completion of reaction, the reaction liquid was analyzed by gas chromatography using analysis column 1 (InertCap 1) to confirm a reaction liquid containing 78.7 g of the target compound represented by the formula (1a) (rate of substrate conversion: 100% (= (Starting compound charged - Remaining starting compound) / Starting compound charged) × 100%) with a dimethanol yield of 96.0% (= Main product / Aldehyde charged × 100%)).

Further, the reaction liquid was subjected to simple distillation purification using a 300 mL three-neck recovery flask equipped with a thermometer, a nozzle for $N_2$ bubbling, and a stirring bar (the number of distillation stages: one stage, distillate temperature: 170 to 180°C, degree of vacuum: 270 Pa (2 Torr)).

The purified product was analyzed by gas chromatography using analysis column 1 (InertCap 1) to confirm 71.6 g of a main fraction containing the target compound represented by the formula (1a) with a dimethanol purity of 99.1% (= Target compound represented by the formula (1a) / (All the components except for acetone) × 100)) and a distillation yield of 91.0% (= Target compound obtained / Target compound charged × 100%)). Also, analysis was conducted using analysis column 2 (InertCap WAX). The amount of the intermediate represented by the formula (3a) was 0.43% (= Intermediate represented by the formula (3a) / (All the components except for 2-octanol and acetone) × 100).

Since the dehydration operation was performed, the effect of reducing the amount of the intermediate represented by the formula (3a) was high even though the amount of the catalyst was reduced, and the amount of the intermediate in the product was less than 0.5%.

Subsequently, transesterification reaction was performed in the same manner as in Example 1 to obtain a polycarbonate resin except that instead of the main fraction obtained in Example 1, the main fraction obtained as described above was used in the same amount as in Example 1. The obtained polycarbonate resin had a weight average molecular weight (Mw) of 7,850 and a glass transition temperature (Tg) of 109°C.

<Example 3>

Reaction was performed by the same operation as in Example 2 except that: the nozzle for nitrogen bubbling was not placed; and the gas-phase moiety was purged four times from 0.5 MPa to 0 MPa. The amount of a liquid trapped was 1.08 g, and the water content therein was 0.34 g. The water concentration of the reaction liquid was 4354 ppm. The amount of the ester compound represented by the formula (3a) 25 hours after the start of the hydrogenation reaction was 0.48%.

After the completion of reaction, the reaction liquid was analyzed by gas chromatography using analysis column 1 (InertCap 1) to confirm a reaction liquid containing 78.7 g of the target compound represented by the formula (1a) (rate of substrate conversion: 100% (= (Starting compound charged - Remaining starting compound) / Starting compound charged) × 100%) with a dimethanol yield of 95.0% (= Target compound / Starting compound charged × 100%)).

Further, the reaction liquid was subjected to simple distillation purification using a 300 mL three-neck recovery flask equipped with a thermometer, a nozzle for $N_2$ bubbling, and a stirring bar (the number of distillation stages: one stage, distillate temperature: 170 to 180°C, degree of vacuum: 2 torr). The purified product was analyzed by gas chromatography using analysis column 1 (InertCap 1) to confirm 71.5 g of a main fraction containing the target compound represented by the formula (1a) with a dimethanol purity of 99.0% (= Target compound represented by the formula (1a) / (All the components except for acetone) × 100)) and a distillation yield of 90.0% (= Target compound obtained / Target compound charged × 100%)). Also, analysis was conducted using analysis column 2 (InertCap WAX). The amount of the intermediate of the formula (3a) was 0.46% (= Intermediate represented by the formula (3a) / (All the components except for 2-octanol and acetone) × 100).

Since the dehydration operation was performed and the water concentration of the reaction liquid was 4500 ppm or less, the effect of reducing the amount of the intermediate represented by the formula (3a) was high, and the amount of the intermediate in the product was less than 0.5%.

Subsequently, transesterification reaction was performed in the same manner as in Example 1 to obtain a polycarbonate resin except that instead of the main fraction obtained in Example 1, the main fraction obtained as described above was used in the same amount as in Example 1. The obtained polycarbonate resin had a weight average molecular weight (Mw) of 7,800 and a glass transition temperature (Tg) of 109°C.

The reaction conditions, the reaction results, etc. of Examples 1 to 3 and Comparative Example 1 are shown in Table 1.

[Table 1]

| | | | Example 1 | Comparative Example 1 | Example 2 | Example 3 |
|---|---|---|---|---|---|---|
| Hydrogenation reaction conditions | Temperature | (°C) | 215 | 215 | 215 | 215 |
| | Pressure | (MPa) | 9.5 | 9.5 | 9.5 | 9.5 |
| | Amount of aldehyde | (g) | 91.6 | 91.6 | 91.6 | 91.6 |
| | Amount of catalyst E01-X | (g) | 15.0 | 15.0 | 9.16 | 9.16 |
| | Catalyst/substrate | (wt%) | 16.4 | 16.4 | 10.0 | 10.0 |
| | Amount of 2-octanol | (g) | 199.4 | 199.4 | 199.4 | 199.4 |

(continued)

| | | | Example 1 | Comparative Example 1 | Example 2 | Example 3 |
|---|---|---|---|---|---|---|
| Water content | Expected water content | (g) | 1.56 | 1.56 | 0.95 | 0.95 |
| | Amount of liquid trapped | | 5.05 | - | 3.01 | 1.08 |
| | Amount of water trapped | | 1.41 | - | 0.85 | 0.34 |
| | Water content of reaction liquid | ppm | 2743 | 7065 | 2585 | 4354 |
| Intermediate content | 5 hours later | (GC%) | 2.20 | 39.91 | 17.97 | 22.60 |
| | 10 hours later | | 0.52 | 13.62 | 5.18 | 6.50 |
| | 15 hours later | | 0.36 | 4.61 | 1.15 | 1.45 |
| | 20 hours later | | | 2.10 | 0.65 | 1.00 |
| | 25 hours later | | | 1.00 | 0.44 | 0.48 |
| Hydrogenation yield | | (mol%) | 96.5 | 94.5 | 96.0 | 95.0 |
| Distillation | Main fraction | (g) | 72.8 | 69.0 | 71.6 | 71.5 |
| | Purity | (GC%) | 99.2 | 98.9 | 99.1 | 99.0 |
| | Distillation vield | (mol%) | 92 | 89 | 91 | 90 |
| | Intermediate content | (GC%) | 0.35 | 0.98 | 0.43 | 0.46 |
| Polycarbonate resin | Molecular weight (Mw) | | 8000 | 6800 | 7850 | 7800 |
| | Glass transition temperature (Tg) | °C | 110 | 105 | 109 | 109 |

The present application claims priority to Japanese Patent Application No. 2021-067254 filed on April 12, 2021.

Industrial Applicability

The production method according to the present invention can produce a dimethanol compound having a norbornane skeleton which is useful as a paint additive, an adhesive, a resin raw material, or the like by an industrially advantageous method.

## Claims

1. A production method for producing a target compound represented by the following formula (1), the production method comprising:

a step (A) of subjecting a mixed liquid comprising a starting compound represented by the following formula (2) and a solvent to a hydrogenation reduction in a presence of a catalyst having hydrogenation ability,
wherein, in the step (A), water is removed from a reaction system and a water content of the mixed liquid measured after the removal of water is 4500 ppm or less:

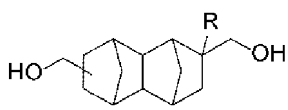

(1)

wherein R represents H, $CH_3$, or $C_2H_5$,

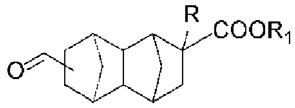

(2)

wherein R represents H, $CH_3$, or $C_2H_5$, and $R_1$ represents $CH_3$, $C_2H_5$, $C_3H_7$, or $C_4H_9$.

2. The production method according to claim 1, wherein, in the step (A), the catalyst is activated at a first temperature and a first pressure, and after the activation, the starting compound is hydrogenated at a second temperature and a second pressure.

3. The production method according to claim 2, wherein, before the hydrogenation, the removal of water is performed.

4. The production method according to claim 2 or 3, wherein the first temperature and

   the first pressure are 100°C or higher and 170°C or lower and 0.5 MPa or higher and 5 MPa or lower, respectively, and
   the second temperature and the second pressure are higher than 170°C and 250°C or lower and higher than 5 MPa and 20 MPa or lower, respectively.

5. The production method according to any one of claims 1 to 4, wherein a content of an intermediate represented by the following formula (3) in the target compound is 0.5% by mass or less:

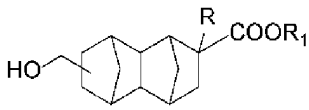

(3)

wherein R and $R_1$ are as defined in the formula (2).

## Patentansprüche

1. Ein Herstellungsverfahren zur Herstellung einer Zielverbindung, dargestellt durch die folgende Formel (1), wobei das Herstellungsverfahren umfasst:

   einen Schritt (A) des Unterziehens eines Flüssigkeitsgemischs, das eine durch die folgende Formel (2) dargestellte Ausgangsverbindung und ein Lösungsmittel umfasst,
   einer Hydrierungsreduktion in Gegenwart eines Katalysators mit Hydrierungsfähigkeit, wobei in dem Schritt (A) Wasser aus einem Reaktionssystem entfernt wird und ein Wassergehalt des Flüssigkeitsgemischs, gemessen nach der Entfernung von Wasser, 4500 ppm oder weniger beträgt:

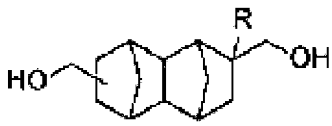

(1)

   wobei R für H, $CH_3$ oder $C_2H_5$ steht,

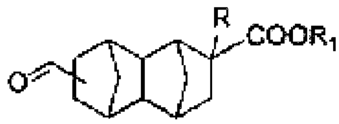

(2)

wobei R für H, $CH_3$ oder $C_2H_5$ steht und $R_1$ für $CH_3$, $C_2H_5$, $C_3H_7$ oder $C_4H_9$ steht.

**2.** Das Herstellungsverfahren nach Anspruch 1, wobei in dem Schritt (A) der Katalysator bei einer ersten Temperatur und einem ersten Druck aktiviert wird und nach der Aktivierung die Ausgangsverbindung bei einer zweiten Temperatur und einem zweiten Druck hydriert wird.

**3.** Das Herstellungsverfahren nach Anspruch 2, wobei vor der Hydrierung die Entfernung von Wasser durchgeführt wird.

**4.** Das Herstellungsverfahren nach Anspruch 2 oder 3, wobei die erste Temperatur und der erste Druck 100°C oder höher und 170°C oder niedriger bzw. 0,5 MPa oder höher und 5 MPa oder niedriger sind und
die zweite Temperatur und der zweite Druck höher als 170°C und 250°C oder niedriger bzw. höher als 5 MPa und 20 MPa oder niedriger sind.

**5.** Das Herstellungsverfahren nach einem der Ansprüche 1 bis 4, wobei ein Gehalt eines durch die folgende Formel (3) dargestellten Zwischenprodukts in der Zielverbindung 0,5 Massen-% oder weniger beträgt:

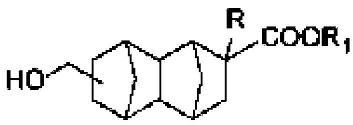

(3)

wobei R und $R_1$ wie in der Formel (2) definiert sind.

## Revendications

**1.** Méthode de production pour produire un composé cible représenté par la formule (1) qui suit, la méthode de production comprenant :

une étape (A) de soumission d'un liquide mixte comprenant un composé de départ représenté par la formule (2) qui suit et un solvant à une réduction avec hydrogénation en présence d'un catalyseur ayant une capacité d'hydrogénation,
dans laquelle, dans l'étape (A), de l'eau est éliminée du système réactionnel et la teneur en eau du liquide mixte, mesurée après l'élimination de l'eau, est de 4 500 ppm ou moins :

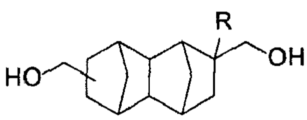

(1)

dans laquelle R représente H, $CH_3$ ou $C_2H_5$,

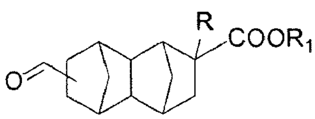

(2)

dans laquelle R représente H, $CH_3$ ou $C_2H_5$, et $R_1$ représente $CH_3$, $C_2H_5$, $C_3H_7$ ou $C_4H_9$.

2. Méthode de production selon la revendication 1, dans laquelle, dans l'étape (A), le catalyseur est activé à une première température et sous une première pression, et après l'activation, le composé de départ est hydrogéné à une deuxième température et sous une deuxième pression.

3. Méthode de production selon la revendication 2, dans laquelle, avant l'hydrogénation, l'élimination de l'eau est effectuée.

4. Méthode de production selon la revendication 2 ou 3, dans laquelle la première température et la première pression sont respectivement de 100 °C ou plus et 170 °C ou moins et de 0,5 MPa ou plus et 5 MPa ou moins, et
la deuxième température et la deuxième pression sont respectivement supérieure à 170 °C et de 250 °C ou moins et supérieure à 5 MPa et de 20 MPa ou moins.

5. Méthode de production selon l'une quelconque des revendications 1 à 4, dans laquelle la teneur en un intermédiaire représenté par la formule (3) qui suit dans le composé cible est de 0,5 % en masse ou moins :

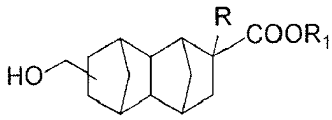

(3)

dans laquelle R et $R_1$ sont tels que définis à propos de la formule (2).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015147242 A
- JP 61161146 A
- JP 1305042 A
- JP 2021067254 A